# EUROPÄISCHE PATENTSCHRIFT

(11) **EP 1 852 140 B1**
(45) Veröffentlichungstag und Bekanntmachung des Hinweises auf die Patenterteilung: **02.12.2009**
(21) Anmeldenummer: 06113359.1
(22) Anmeldetag: 02.05.2006
(51) Int. Cl.: A61M 25/02

(54) **Schnellbefestigungsvorrichtung für Katheter**
Fast anchorage apparatus for catheter
Dispositif de fixation rapide pour cathéter

(43) Veröffentlichungstag der Anmeldung: 07.11.2007
(73) Patentinhaber: Pulsion Medical Systems AG, 81829 München (DE)
(72) Erfinder: Goedje, Dr. Oliver, 82031 Grünwald (DE); Thalmeier, Thomas, 84405 Dorfen (DE); Diethelm, Holger, 81475 München (DE)
(74) Vertreter: Ettmayr, Andreas

(56) Entgegenhaltungen:
- EP-A- 0 116 526
- WO-A-00/54830
- WO-A-93/16751
- WO-A-98/25508
- WO-A-02/068024
- WO-A-03/051447
- US-A- 5 267 970

## Beschreibung

Die vorliegende Erfindung betrifft eine Schnellbefestigungsvorrichtung für Katheter, insbesondere intravasale Katheter.

Intravasale Katheter sind in der Medizin, insbesondere der Intensivmedizin in vielfältiger Verwendung. Als Gefäßzugang dienen sie der Zuführung von Stoffen in die Blutbahn, der Einführung von Meßssonden, Entnahme von Proben sowie der Einrichtung von Druck- und Temperaturmeßstellen. Häufig besitzt ein Katheter mehr als ein Lumen, um verschiedene Funktionen ausüben zu können (beispielsweise Blutdruckmessung und Blutprobenentnahme). Distal von einer als Kanaltrennung bezeichneten Verzweigung verlaufen diese Lumen in einem gemeinsamen Katheterkörper, proximal von der Kanaltrennung verlaufen die Lumen in separaten Fortsätzen.

Zum Legen eines intravasalen Katheters wird das bestimmungsgemäße Gefäß punktiert und anschließend der Katheter, je nach Anwendung beispielsweise nach Einführung eines Führungsdrahts und Dilation (Weitung) der Einstichstelle nach der sogenannten Seldingertechnik, eingeführt. Da der Katheter in der Regel über längere Zeit, meist über Tage oder zumindest Stunden im Gefäß verbleibt, ist es erforderlich, diesen am Patienten zu fixieren, um zu vermeiden, daß sich - etwa bei Bewegungen des Patienten - die Position des Katheters im Gefäß verändert: Eine Bewegung des Katheters weiter aus dem Gefäß heraus hätte zur Folge, daß die am distalen Katheterende befindlichen Öffnungen, Sensormeßstellen etc. ihre Funktion nicht mehr am ursprünglich vorgesehenen Ort ausüben; eine Bewegung des Katheters weiter in das Gefäß hinein birgt zudem die Gefahr von Infektionen. Darüberhinaus würde eine übermäßige Bewegung des Katheters am Ort seines Durchtritts durch das Gewebe des Patienten dieses weiter verletzen. Vor allem ist jedoch zu vermeiden, daß der Katheter ganz aus dem Gefäß herausgezogen wird. Gerade bei einem arteriellen Katheter würde dies erheblichen Blutverlust an der Punktionsstelle bedeuten.

Üblicherweise werden Katheter angenäht, um sie am Patienten zu fixieren. Häufig sind hierfür im Bereich der Kanaltrennung des Katheters, worunter wie gesagt der weichenartige Übergang zwischen dem distalen Katheterkörper und zwei oder mehreren proximalen Katheteranschlüssen verstanden wird, Ösen vorgesehen, durch welche der Faden gezogen wird. Beispielsweise zeigt die Offenlegungsschrift WO 00/54830 A1 eine Katheterkanaltrennung, die zur Fixierung der Vorrichtung Öffnungen für eine chirurgische Naht aufweist.

Für das Annähen muss Zeit aufgewendet werden, welche die weitere medizinische Versorgung verzögern kann. Dies ist gerade bei Notfallpatienten problematisch. Zum Nähen benötigt man zwei Hände, was unter Umständen dazu führen kann, dass eine zusätzliche Hilfsperson erforderlich wird, um gleichzeitig andere wichtige Handgriffe vorzunehmen. Ferner muss steriles Nahtmaterial bereitgehalten werden.

Bislang wurde der Aufwand an Zeit und Nahtmaterial beim Legen eines intravasalen Katheters als unvermeidlich angesehen.

Die Offenlegungsschrift WO 03/051447 A1 beschreibt eine Vorrichtung zum Befestigen bzw. zum Haltern eines schlauchartigen Mittels an einem Körperteil, vorzugsweise an der Hautoberfläche, eines Lebewesens. Dabei wird ein sogenannter 'Fixationsclip' auf einen bereits in die Haut eingeführten Drainage- oder Infusionsschlauch aufgeklemmt.

Die Offenlegungsschrift EP 0 116 536 A1 beschreibt eine Vorrichtung zum Fixieren von Kathetern, Drainagen und Schläuchen an der Haut. Die Vorrichtung weist Befestigungsmittel und mindestens ein Fixiermittel, das am Befestigungsmittel anschließbar ist, auf. Das Fixiermittel ist am Befestigungsmittel beweglich und fixierbar angebracht und beispielsweise als Nadel oder als bogenförmiger Haken ausgebildet.

Die Offenlegungsschrift WO 9316751 A offenbart eine Vorrichtung, die ein erstes und ein zweites Befestigungsmittel aufweist, die bei Ingebrauchnahme aktiviert werden, um einen Schlauch zu haltern und die Vorrichtung an der Haut des Patienten zu fixieren.

Die Offenlegungsschrift US 5,267,970 A offenbart eine Vorrichtung mit rohrförmiger Hülse, eine Haltevorrichtung zur Halterung der Hülse und ein Halterung zur Fixierung der Vorrichtung mittels Klammerung an der Haut des Patienten.

Die Offenlegungsschrift WO 02/068024 A1 zeigt eine Vorrichtung zur Fixierung eines Katheters an einen Patienten. Die Fixierung erfolgt mittels bogenförmiger Nadeln, die sich bei Bedarf in der Haut des Patienten verfangen. Das Haltern der Vorrichtung setzt eine ebene Hautoberfläche voraus.

Die Offenlegungsschrift WO 98/25508 A offenbart einen chirurgischen Heftapparat. Durch Zusammendrücken der Vorrichtung wird ein innenliegender Bügel, der einen Hauptstrang und zwei identische Arme aufweist, so gestaucht, dass die Arme die Vorrichtung am Patienten fixieren.

Angesichts der obengeschilderten Problematik liegt der Erfindung die Aufgabe zugrunde, eine Beschleunigung der Katheterfixierung zu ermöglichen, wobei die Schnellbefestigung sicher und unverrückbar an der Kanaltrennung des Katheters gehalten ist.

Gemäß einem Aspekt der Erfindung wird diese Aufgabe durch eine Schnellbefestigungsvorrichtung für Katheter nach Anspruch 1 und Anspruch 13 gelöst.

Vorteilhafte Ausführungsformen der Erfindung können gemäß einem der Ansprüche 2 - 12 gestaltet sein.

Haltemittel der mit einem zu fixierenden Katheter verbundenen Schnellbefestigungsvorrichtung werden ausgelenkt und am Patienten angelegt. Durch Zurückbewegen der Haltemittel in eine weniger starkausgelenkte Position wird eine form- und/oder kraftschlüssige Verbindung, beispielsweise durch Einhaken in der Haut, zwischen Patient und Schnellbefestigungsvorrichtung geschaffen. Eine durch geeignete Mittel aufgebrachte Gegenkraft verhindert, dass die Haltemittel nach dem Fixieren unbeabsichtigt wieder so weit ausgelenkt werden, dass sich die Schnellbefestigungsvorrichtung vom Patienten lösen könnte. Vorteilhaft kann dies durch Einrasten oder die Rückstellkraft eingesetzter Federmittel erzielt werden.

Ohne die Verwendung von Nahtmaterial kann so mit wenigen Handgriffen eine sichere Fixierung des Katheters am Patienten erreicht werden. Insbesondere bei klammer- oder zangenartiger Ausführung der Haltemittel lässt sich die vorzugsweise ermöglichte Einhandbedienung umsetzen. Gerade bei der Versorgung von Notfallpatienten erreicht man gegenüber dem Stand der Technik so eine wertvolle Zeitersparnis und eine geringere Ablenkung des versorgenden Arzts oder Pflegepersonals.

Die Schnellbefestigungsvorrichtung kann entweder in die Kanaltrennung eines Katheters integriert sein, oder aber sie ist als separates Bauteil ausgeführt, welches beispielsweise mittels Klammern, Haken, Federzungen, Schlaufen oder dergleichen mit dem zu fixierenden Katheter verbindbar ist.

Grundsätzlich kann jede im Rahmen der vorliegenden Anmeldung beschriebene bzw. angedeutete Variante der Erfindung besonders vorteilhaft sein, je nach wirtschaftlichen und technischen Bedingungen im Einzelfall. Soweit nichts gegenteiliges dargelegt ist, bzw. soweit grundsätzlich technisch realisierbar, sind einzelne Merkmale der beschriebenen Ausführungsformen austauschbar oder miteinander kombinierbar.

Nachfolgend werden anhand der zugehörigen Zeichnungen Beispiele bevorzugter Ausführungsformen der vorliegenden Erfindung näher erläutert.

Die Zeichnungen sind dabei rein schematische und, aus Gründen der Anschaulichkeit, nicht maßstäbliche Darstellungen. Insbesondere können Verhältnisse der Abmessungen zueinander von tatsächlichen Ausführungen abweichen.

Einander entsprechende Elemente sind in den einzelnen Figuren jeweils, soweit sinnvoll, mit den gleichen Bezugszeichen versehen.
- Fig. 1: zeigt schematisch in der Draufsicht den Bereich der Kanaltrennung eines Katheters mit einer erfindungsgemäßen, klammerartig ausgeführten Schnellbefestigungsvorrichtung.
- Fig. 2: zeigt schematisch den Bereich der Kanaltrennung eines Katheters mit einer ähnlich Fig. 1, jedoch als Doppelklammer ausgeführten Schnellbefestigungsvorrichtung.
- Fig. 3a-d: illustrieren die Anwendung der in Fig. 1 dargestellten Schnellbefestigungsvorrichtung.
- Fig. 4: zeigt in perspektivischer Ansicht eine Variante der Verbindung einer ähnlich Fig. 1 ausgeführten Schnellbefestigungsvorrichtung mit der Kanaltrennung eines Katheters.

- Fig. 5: zeigt in perspektivischer Ansicht eine Variante der Integration einer ähnlich Fig. 1 ausgeführten Schnellbefestigungsvorrichtung in die Kanaltrennung eines Katheters.
- Fig. 6: zeigt eine alternative Ausführungsform einer erfindungsgemäßen Schnellbefestigungsvorrichtung, welche mit herkömmlichen, Nahtösen aufweisenden Kanaltrennungen verbindbar ist.
- Fig. 7a: zeigt eine weitere alternative Ausführungsform einer erfindungsgemäßen Schnellbefestigungsvorrichtung in halb ausgelenktem Zustand.
- Fig. 7b: zeigt die Ausführungsform aus Fig. 7a in zurückgestelltem Zustand.
- Fig. 7c: illustriert die Verbindung zwischen der in Fig. 7a und Fig. 7b dargestellten Schnellbefestigungsvorrichtung und der Kanaltrennung eines Katheters in einer seitlichen Ansicht.
- Fig. 8: zeigt schematisch den Bereich der Kanaltrennung eines Katheters gemäß dem Stand der Technik.

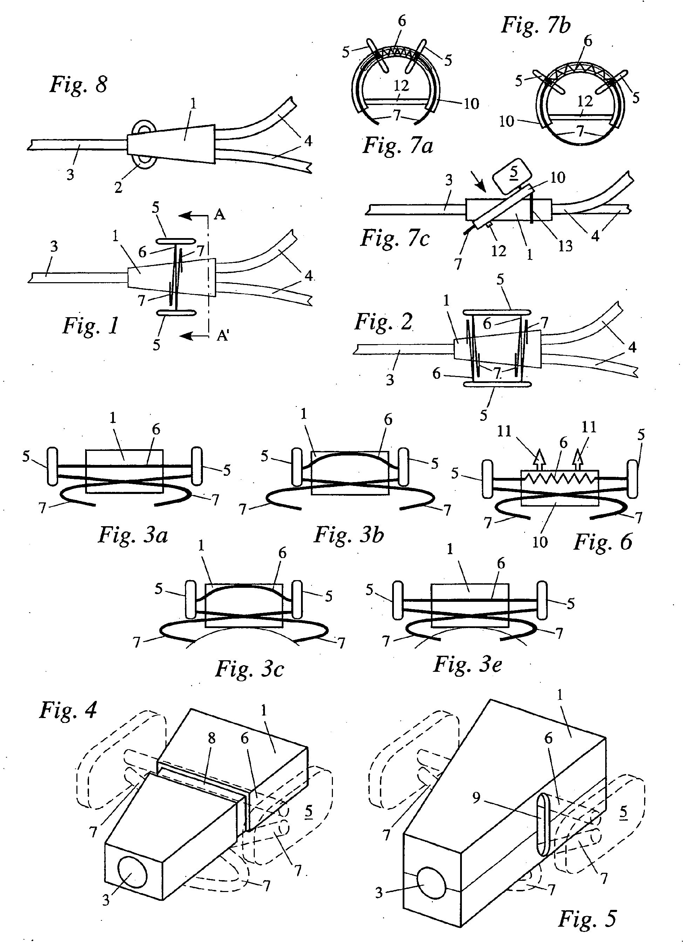

Wie in Fig. 8 dargestellt, sind an der Kanaltrennung 1 eines intravasalen Katheters oft Ösen 2 zum Durchziehen des Fadens vorgesehen, mit welchem der Katheter am Patienten angenäht wird. Der distale Katheterkörper 3 sowie die proximalen Katheteranschlüsse 4 sind nur ausschnittsweise dargestellt.

Die in Figuren 1 und 2 (aus Gründen der Übersichtlichkeit in dünneren Linien angedeutete) Kanaltrennung 1 mit distalem Katheterkörper 3 und proximalen Katheteranschlüssen 4 entspricht im wesentlichen dem Stand der Technik, ist jedoch mit einer erfindungsgemäßen Schnellbefestigungsvorrichtung ausgestattet. Ösen sind in den dargestellten Ausführungsformen nicht vorgesehen; die Schnellbefestigungsvorrichtung kann gemäß einer der in Figuren 4 und 5 dargestellten Varianten mit der Kanaltrennung 1 verbunden sein. Erfindungsgemäße Schnellbefestigungsvorrichtungen können jedoch, wie unten anhand Fig. 6 beschrieben, auch so konstruiert werden, daß sie mit herkömmlichen, Nahtösen aufweisenden Kanaltrennungen verbindbar sind.

Die in Fig. 1 dargestellte Schnellbefestigungsvorrichtung weist zwei Platten mit außenliegenden Druckflächen 5, die in Form eines Biegestabs ausgeführte Feder 6 sowie zwei sterile Edelstahlhaken 7 auf. Statt der Edelstahlhaken 7 können auch Haken aus einem anderen Material oder Materialverbund vorgesehen werden. Feder 6 und Edelstahlhaken 7 bilden zusammen eine Klammer. Dabei kann die Feder 6 beispielsweise eine separat in die beiden Platten eingesetzte Zunge aus Federstahl sein. Feder 6 und Edelstahlhaken 7 können aber auch gemeinsam aus einem Materialstück gebogen sein.

Bei der in Fig. 2 abgebildeten Schnellbefestigungsvorrichtung sind zwei wie in Fig. 1 ausgeführte Klammern vorgesehen, welche gemeinsam (einhändig) über die Druckflächen 5 bedient werden.

Figuren 3a-e illustrieren die Funktionsweise der Schnellbefestigungsvorrichtung aus Fig. 1. Die Darstellungsebene ist dabei um 90 Grad gedreht, wie in Fig. 1 durch die strichpunktierte Linie A-A' angedeutet, wobei die Pfeile die Blickrichtung angeben. Die Schnellbefestigungsvorrichtung aus Fig. 2 funktioniert auf gleiche Weise.

Werden die Druckflächen 5 zueinander gedrückt, so biegt sich die Feder 6, und die sterilen Edelstahlhaken 7 werden aus ihrer ursprünglichen Lage ausgelenkt, wie in Fig. 3b erkennbar. Mit ausgelenkten Edelstahlhaken 7 wird die Schnellbefestigungsvorrichtung, wie in Fig. 3c dargestellt, an die (durch eine dünne gekrümmte Linie angedeutete) Haut des Patienten angelegt. Nach Loslassen der Druckflächen 5 strebt die Feder 6 in ihre Ausgangslage. Durch die Rückstellkraft der Feder 6 haken sich die, zu diesem Zweck an ihren Enden angespitzten, Edelstahlhaken 7 in die Haut des Patienten, wie in Fig. 3e angedeutet.

Figuren 4 und 5 zeigen zwei Beispiele einer Verbindung zwischen der wie in Fig. 1 ausgeführten Klammer einer erfindungsgemäßen Schnellbefestigungsvorrichtung und der Kanaltrennung 1 des zu fixierenden Katheters. Der Ansatz des distalen Katheterkörpers 3 ist nur angedeutet. Wie nach dem Stand der Technik üblich bietet sich an, das Gehäuse der Kanaltrennung 1 als Spritzgussteil auszuführen. Selbstverständlich sind auch andere Fertigungsweisen möglich.

Aus Gründen der Übersichtlichkeit ist die Schnellbefestigungsklammer in Figuren 4 und 5 strichliert gezeichnet.

In Fig. 4 besitzt die Kanaltrennung 1 eine umlaufende Nut 8. Auch wenn diese U-förmig dargestellt ist, kann sie ebensogut keilförmig oder mit gerundetem Querschnitt ausgeführt werden. Die Schnellbefestigungsklammer ist von vorne über die Kanaltrennung 1 geschoben. Die Rückstellkraft der Feder 6 hält diese in der Nut 8, in welcher auch die Haken 7 teilweise zu
liegen kommen, so dass die Kanaltrennung 1 und die Schnellbefestigungsklammer sicher aneinander gehaltert sind.

Bei der in Fig. 5 dargestellten, nicht ganz so flach ausführbaren Variante sind Feder 6 und Haken 7 durch das Spritzgußgehäuse der Kanaltrennung 1 geführt. Zu diesem Zweck besitzt das Spritzgussgehäuse seitliche Schlitze 9 und die Schnellbefestigungsklammer wird bereits bei dessen Fertigung in das Gehäuse eingesetzt.

Auch eine Zwitterversion der in Fig. 4 und Fig. 5 dargestellten Varianten, bei welcher die Edelstahlhaken 7 durch das Spritzgußgehäuse der Kanaltrennung 1 laufen, und die Feder 6 in einer Nut 8 oder auch einfach auf der flachen Oberseite der Kanaltrennung 1 zu liegen kommt, ist möglich. Eine derartige Lösung ermöglicht eine relativ flache Ausführung der Kanaltrennung 1.

In Fig. 6 ist eine weitere Ausführungsform einer erfindungsgemäßen Schnellbefestigungsvorrichtung in einer Fig. 3a analogen Ansicht dargestellt, wobei jedoch auf die Abbildung der Kanaltrennung verzichtet wurde. Anstatt eines Biegestabs ist die Feder 6 als Spiralfeder ausgeführt. Die Schnellbefestigungsvorrichtung besitzt ein eigenes flaches Gehäuse 10, welches nicht mit dem Gehäuse der Kanaltrennung des zu fixierenden Katheters identisch ist. Auf dem Gehäuse 10 sind zwei Dorne 11 angeordnet, welche vorzugsweise aus einem gummielastischen Material bestehen. Die Dorne 11 können beispielsweise in Bohrungen im Gehäuse 10 verankert sein (nicht dargestellt). Mittels der Dorne 11 läßt sich eine wie in Fig. 8 ausgeführte herkömmliche Kanaltrennung 1 mit der Schnellbefestigungsvorrichtung verbinden, indem die Dorne 11 durch deren Ösen 2 gesteckt werden. Anstelle der Dorne 11 können beispielsweise auch einfache Drahtstifte vorgesehen sein, welche nach Durchführen durch die Ösen 2 umgebogen werden.

Figuren 7a, 7b und 7c zeigen eine weitere Ausführungsform einer erfindungsgemäßen Schnellbefestigungsvorrichtung. In Fig. 7a und 7b ist die Schnellbefestigungsvorrichtung ohne Katheter in der Draufsicht, in Fig. 7c mit Katheter in einer seitlichen Ansicht dargestellt. Der Pfeil in Fig. 7c deutet die Blickrichtung von Fig. 7a und 7b an.

In das teilringförmige Gehäuse 10 sind zwei Haken 7 eingesetzt, deren im Gehäuse 10 liegenden Enden von der Feder 6 auseinandergedrückt werden. Entsprechend werden die außerhalb des Gehäuses 10 liegenden Enden der Haken 7 von der Feder 6 zueinandergedrückt (= rückgestellt).

Bei Zusammendrücken der Feder 6 mittels zweier an den im Gehäuse 10 liegenden Enden der Haken 7 angesetzten Platten streben die außerhalb des Gehäuses 10 liegenden Enden der Haken 7 auseinander (werden ausgelenkt). Somit läßt sich die Schnellbefestigungsvorrichtung durch Druck auf die Druckflächen 5 der Platten bedienen wie die Schnellbefestigungsvorrichtung aus Fig. 1.

Fig. 7a zeigt die Schnellbefestigungsvorrichtung in halb ausgelenktem, Fig 7b im vollständig rückgestellten Zustand. Die Feder 6 ist so ausgewählt, daß sich die beiden außerhalb des Gehäuses 10 liegenden Enden bei der Anwendung im Gewebe des Patienten treffen und sozusagen einen Ringschluß bilden. Dies hat den Vorteil, daß keine freien Enden unkontrolliert Gewebe traumatisieren können und bei Bewegung "pieksen". Auch bei anderen Ausführungen können die Abmessungen vorteilhafterweise so gewählt werden, daß die im Patientengewebe befindlichen Enden von Hakmitteln aufeinandertreffen.

Die Kanaltrennung 1 des zu fixierenden Katheters ist mittels einer einfachen Klemmung am Gehäuse 10 befestigt. Sie liegt auf der Traverse. 12 auf und wird von einer elastischen Schlaufe (z.B. aus Gummi) oder Zunge 13 (z.B. aus Federstahl) gehaltert, welche die Kanaltrennung 1 an das teilringförmige Gehäuse 10 heranzieht.

## Patentansprüche

1. Schnellbefestigungsvorrichtung für Katheter, aufweisend
- auslenkbare Haltemittel (7) zum Halten des Katheters an einem Patienten,
- Mittel (6), welche zumindest in einer Position der Haltemittel (7) eine Gegenkraft entgegen einer weiteren Auslenkung der Haltemittel ausüben
**dadurch gekennzeichnet, daß** die Schnellbefestigungsvorrichtung in eine Katheterkanaltrennung (1) integriert ist.

2. Schnellbefestigungsvorrichtung gemäß Anspruch 1, wobei die Haltemittel (7) Hakmittel (7) zum Einhaken in die Haut des Patienten aufweisen.

3. Schnellbefestigungsvorrichtung gemäß Anspruch 2, wobei die Hakmittel (7) aus Edelstahl ausgeführt sind.

4. Schnellbefestigungsvorrichtung gemäß einem der Ansprüche 2-3, wobei die Hakmittel (7) aus Kunststoff ausgeführt oder mit Kunststoff ummantelt oder beschichtet sind.

5. Schnellbefestigungsvorrichtung gemäß einem der Ansprüche 2-4, wobei die Hakmittel (7) steril sind.

6. Schnellbefestigungsvorrichtung gemäß einem der vorangehenden Ansprüche, wobei die Mittel (6), welche zumindest in einer Position der Haltemittel eine Gegenkraft entgegen einer weiteren Auslenkung der Haltemittel (7) ausüben, Einrastmittel umfassen.

7. Schnellbefestigungsvorrichtung gemäß einem der vorangehenden Ansprüche, wobei die Mittel (6), welche zumindest in einer Position der Haltemittel eine Gegenkraft entgegen einer weiteren Auslenkung der Haltemittel ausüben, Federmittel (6) umfassen, welche eine Rückstellkraft auf die Haltemittel (7) ausüben, wenn sich die Haltemittel (7) im ausgelenkten Zustand befinden.

8. Schnellbefestigungsvorrichtung gemäß Anspruch 7, aufweisend Klammermittel, welche Haltemittel (7) und Federmittel (6) umfassen.

9. Schnellbefestigungsvorrichtung gemäß Anspruch 8, wobei die Haltemittel (7) durch Spreizen der Klammermittel auslenkbar sind.

10. Schnellbefestigungsvorrichtung gemäß einem der vorangehenden Ansprüche, bei welcher die Haltemittel (7) einhändig auslenkbar sind.

11. Schnellbefestigungsvorrichtung gemäß Anspruch 10, aufweisend zwei zueinander drückbare Druckflächen (5) zum Auslenken der Haltemittel (7).

12. Katheter, welcher mit einer Schnellbefestigungsvorrichtung gemäß einem der vorangehenden Ansprüche ausgestattet ist.

13. Anordnung, welche einen Katheter mit einer Kanaltrennung und eine Schnellbefestigungsvorrichtung, aufweisend
- auslenkbare Haltemittel (7) zum Halten des Katheters an einem Patienten,
- Mittel (6), welche zumindest in einer Position der Haltemittel (7) eine Gegenkraft entgegen einer weiteren Auslenkung der Haltemittel ausüben, umfaßt,
**dadurch gekennzeichnet, dass** die Kanaltrennung eine umlaufende Nut (8) zur Anbringung der Schnellbefestigungsvorrichtung an der Katheterkanaltrennung (1) aufweist, und die Schnellbefestigungsvorrichtung zum Haltern in der umlaufenden Nut (8) der Katheterkanaltrennung (1) angepasst ist.

## Claims

1. A quick-attachment device for catheters, comprising:
deflectable holding means (7) for holding the catheter on a patient; and
means (6) that exert a counter-force counter to further outward deflection of the holder, at least in one position of the holding means (7),
**characterised in that** the quick-attachment device is integrated into a catheter channel separation (1).

2. A quick-attachment device according to claim 1, wherein said at least one holding means (7) comprises hooking means (7) for hooking into the skin of the patient.

3. A quick-attachment device according to claim 2, wherein the hooking means (7) are made from stainless steel.

4. A quick-attachment device according to any one of claims 2-3, wherein the hooking means (7) are made from plastic, or are mantled with plastic, or are coated with plastic.

5. A quick-attachment device according to any one of claims 2-4, wherein the hooking means (7) are sterile.

6. A quick-attachment device according to any one of the preceding claims, wherein the means (6) that exert a counter-force counter to further deflection of the holding means (7), comprise engagement means.

7. A quick-attachment device according to any one of the preceding claims, wherein the means (6) that exert a counter-force counter to further deflection of the holding means (7), comprise spring means (6) that exert a re-set force on the holding means (7) when the holding means (7) is in the deflected state.

8. A quick-attachment device according to claim 7, further comprising clamping means, that include the holding means (7) and spring means (6).

9. A quick-attachment device according to claim 8, wherein the holding means (7) can be deflected by spreading the clamping means.

10. A quick-attachment device according to any one of the preceding claims, wherein the holding means (7) can be deflected with one hand.

11. A quick-attachment device according to claim 10, further comprising two push surfaces (5) that can be pressed toward one another, for deflecting the holding means (7).

12. A catheter that is equipped with a quick-attachment device according to any one of the preceding claims.

13. An arrangement comprising a catheter having a channel separation and a quick-attachment device, comprising:
deflectable holding means (7) for holding the catheter on a patient; and
means (6) that exert a counter-force counter to further outward deflection of the holder, at least in one position of the holding means (7),
**characterised in that** the channel separation comprises a circumferential groove (8) for affixing the quick-attachment device to the catheter channel separation (1), and
the quick-attachment device is adapted for holding in the groove (8) of the catheter channel separation (1).

## Revendications

1. - Dispositif de fixation rapide pour cathéters, comprenant :
- des moyens de retenue (7) pouvant subir une flexion pour la retenue du cathéter sur un patient ;
- des moyens (6), lesquels exercent, au moins dans une position des moyens de retenue (7), une force antagoniste à l'encontre d'une nouvelle flexion des moyens de retenue,
**caractérisé par le fait que** le dispositif de fixation rapide est intégré dans une séparation des canaux de cathéter (1).

2. - Dispositif de fixation rapide selon la revendication 1, suivant lequel les moyens de retenue (7) comprennent des moyens d'accrochage (7) pour l'accrochage dans la peau du patient.

3. - Dispositif de fixation rapide selon la revendication 2, suivant lequel les moyens d'accrochage (7) sont réalisés en acier inoxydable.

4. - Dispositif de fixation rapide selon l'une des revendications 2 et 3, suivant lequel les moyens d'accrochage (7) sont réalisés en matière plastique ou sont gainés ou revêtus par une matière plastique.

5. - Dispositif de fixation rapide selon l'une des revendications 2 à 4, suivant lequel les moyens d'accrochage (7) sont stériles.

6. - Dispositif de fixation rapide selon l'une des revendications précédentes, suivant lequel les moyens (6), lesquels exercent au moins dans une position des moyens de retenue une force antagoniste à l'encontre d'une nouvelle flexion des moyens de retenue (7), comprennent des moyens d'encliquetage.

7. - Dispositif de fixation rapide selon l'une des revendications précédentes, suivant lequel les moyens (6), lesquels exercent au moins dans une position des moyens de retenue une force antagoniste à l'encontre d'une nouvelle flexion des moyens de retenue, comprennent des moyens élastiques (6), lesquels exercent une force de rappel sur les moyens de retenue (7), lorsque les moyens de retenue (7) se trouvent dans un état fléchi.

8. - Dispositif de fixation rapide selon la revendication 7, présentant des moyens de serrage, lesquels comprennent des moyens de retenue (7) et des moyens élastiques (6).

9. - Dispositif de fixation rapide selon la revendication 8, suivant lequel les moyens de retenue (7) sont aptes à subir une flexion par écartement des moyens de serrage.

10. - Dispositif de fixation rapide selon l'une des revendications précédentes, suivant lequel les moyens de retenue (7) sont aptes à subir une flexion d'une main.

11. - Dispositif de fixation rapide selon la revendication (10), présentant deux surfaces de pression (5) pouvant être pressées l'une contre l'autre pour la flexion des moyens de retenue (7).

12. - Cathéter, qui est équipé d'un dispositif de fixation rapide selon l'une des revendications précédentes.

13. - Dispositif, lequel comprend un cathéter avec une séparation de canaux et un dispositif de fixation rapide, comprenant :
- des moyens de retenue (7) pouvant subir une flexion pour la retenue du cathéter sur un patient ;
- des moyens (6), lesquels exercent au moins dans une position des moyens de retenue (7) une force antagoniste à l'encontre d'une nouvelle flexion des moyens de retenue,
**caractérisé par le fait que** la séparation des canaux présente une rainure périphérique (8) pour l'application du dispositif de fixation rapide sur la séparation des canaux de cathéter (1),
et le dispositif de fixation rapide est adapté pour la retenue dans la rainure périphérique (8) de la séparation des canaux de cathéter (1).
